# EUROPÄISCHE PATENTSCHRIFT

(11) **EP 1 297 106 B1**
(45) Veröffentlichungstag und Bekanntmachung des Hinweises auf die Patenterteilung: **10.11.2004**
(21) Anmeldenummer: 01940329.4
(22) Anmeldetag: 14.04.2001
(51) Int. Cl.: C12M 3/06

(54) **REAKTORMODUL MIT KAPILLARMEMBRANEN**
REACTOR MODULE WITH CAPILLARY MEMBRANES
MODULE REACTEUR A MEMBRANES CAPILLAIRES

(30) Priorität: 13.05.2000 DE 10023505
(43) Veröffentlichungstag der Anmeldung: 02.04.2003
(73) Patentinhaber: Fraunhofer-Gesellschaft zur Förderung der angewandten Forschung e.V., 80686 München (DE)
(72) Erfinder: STROH, Norbert, 71106 Magstadt (DE); GRAEVE, Thomas, 70372 Stuttgart (DE)
(86) Internationale Anmeldenummer: PCT/EP2001/004271
(87) Internationale Veröffentlichungsnummer: WO 2001/088083

(56) Entgegenhaltungen:
- WO-A-01/09607
- WO-A-84/01959
- WO-A-90/02170
- DE-A- 3 637 260
- GB-A- 2 159 729
- US-A- 5 595 909
- US-A- 5 605 835
- US-A- 5 622 857
- US-A- 6 001 585
- PATENT ABSTRACTS OF JAPAN vol. 013, no. 169 (C-587), 21. April 1989 (1989-04-21) & JP 63 317076 A (NOK CORP), 26. Dezember 1988 (1988-12-26)
- PATENT ABSTRACTS OF JAPAN vol. 013, no. 169 (C-587), 21. April 1989 (1989-04-21) & JP 63 317075 A (NOK CORP), 26. Dezember 1988 (1988-12-26)
- PATENT ABSTRACTS OF JAPAN vol. 017, no. 216 (C-1053), 28. April 1993 (1993-04-28) & JP 04 356184 A (TABAI ESPEC CORP), 9. Dezember 1992 (1992-12-09)
- PATENT ABSTRACTS OF JAPAN vol. 013, no. 362 (C-625), 14. August 1989 (1989-08-14) & JP 01 119309 A (TOKYO INST OF TECHNOL), 11. Mai 1989 (1989-05-11)

## Beschreibung

Die vorliegende Erfindung betrifft ein Reaktormodul, welches als Bestandteil eines künstlichen Organes dienen kann sowie einen dieses Reaktormodul enthaltenden Reaktor.

Erkrankungen insbesondere innerer Organe des Menschen sind häufig und oft lebensbedrohend. Die oftmals notwendigen Transplantationen von Organen scheitern häufig an der nur begrenzten Verfügbarkeit von natürlichen Ersatzorganen. Externe Systeme zur Unterstützung der beeinträchtigten oder ausgefallenen Organfunktionen erweisen sich in vielen Fällen als unzureichend, weil sie einerseits die Lebensqualität des Patienten erheblich herabsetzen und überdies in die biochemischen Abläufe des Körpers nicht eingreifen können. Am Beispiel der Leber wurden in den letzten Jahren hybride Leberunterstützungssysteme entwickelt, gemäß denen Leberzellen in künstlichen Modulen kultiviert werden und wobei verschiedene Metabolisierungs-, Eliminations- und Synthesevorgänge der Leber bei Patienten mit akutem Leberversagen ersetzt werden können. Die vorhandenen Systeme basieren auf einem extrakorporalen Kreislauf, an den der Patient angeschlossen wird. Der verwendete Reaktor besteht aus einem Gehäuse, in dem sich die Leberzellen befinden. Das Patientenblut beziehungsweise Plasma steht in direktem oder indirektem Kontakt zu den Zellen. Es ist dabei bekannt, die Hepatozyten in oder auf kleinen Kugeln zu immobiliseren, zum Beispiel in Alginat (Selden et al., Ann N Y Acad Sci (1999) 875, 353-363; Naka et al., Artificial Organs (1999) 23, 822-828 und Sakai et al., Cell Transplantation (1999) 8, 531-541). Bekannt ist es auch, das parenchymale Gefüge der Leber mittels mehrdimensional angeordneter Kapillarbündel nachzuahmen (Custer und Mullon, Adv Exp Med Biol (1998) 454, 261-271; Busse und Gerlach, Ann N Y Acad Sci (1999) 875, 326-339, Flendrig et al., Int J Artif Organs (1999) 22, 701-708; Margulis et al., Resuscitation (1989) 18, 85-94; Ellis et al., Hepatology (1996) 24, 1446-1451; Gerlach et al., Transplantation (1994) 58, 984-988) .

Die beschriebenen künstlichen Organe, bei denen natürliche Zellen auf einer Unterstruktur, zum Beispiel polymeren Hohlfasern oder Kapillaren, immobilisiert sind, weisen den Nachteil auf, dass die Zellen häufig nur unter Problemen immobilisiert werden können. Dies liegt unter anderem daran, dass mit Durchströmungspulsen einhergehende Durchmesserschwankungen von herkömmlicherweise verwendeten polymeren Hohlfasern die Immobilisierung erschweren und auch zu Denaturierungseffekten der Zellen führen können.

US-A-5,605,835 offenbart einen modularen Flach-Bett Bioreaktor, bei dem biologische Zellen auf einer flachen keramischen Membran, die in einen eckigen Rahmen eingespannt ist, immobilisiert sind. Weiterhin offenbart dieses Dokument einen Bioreaktor mit keramischen Hohlfasern, bei dem die biologische Zellen im extrakapillaren Bereich immobilisiert sind.

Der vorliegenden Erfindung liegt daher das technische Problem zu Grunde, ein Reaktormodul für den Einsatz in künstlichen Organen bereitzustellen, welches die vorgenannten Nachteile überwindet.

Die vorliegende Erfindung löst das ihr zugrundeliegende technische Problem durch die Bereitstellung eines Reaktormoduls gemäß dem Anspruch 1. In besonders bevorzugter Ausführungsform ist die mindestens eine biologische Zelle eine Leberzelle, also eine Hepatozyte. Selbstverständlich können jedoch auch andere Zellen erfindungsgemäß eingesetzt werden, wie Nierenzellen, Bindegewebszellen, Fibroblasten, Immunzellen, Darmzellen, Hautzellen, Zellen aus der Bauchspeicheldrüse, aus der Milz oder Blutzellen.

In einer weiteren bevorzugten Ausführungsform der Erfindung sind auf der Oberfläche der mindestens einen keramischen Hohlfaser viele Zellen, insbesondere eine Zellschicht, insbesondere eine Monolayer, immobilisiert.

Die Erfindung ist unter anderem insofern vorteilhaft, als dass die Eigensteifigkeit der verwendeten keramischen Hohlfasern ihre definierte räumliche Positionierung innerhalb eines Reaktorraums ermöglicht. Im Gegensatz zu polymeren Hohlfasern oder Kapillaren ergeben sich keine mit Durchströmungspulsen einhergehende Durchmesserschwankungen, so dass die immobilisierten Zellen nicht beeinträchtigt werden können. Die keramischen Hohlfasern lassen sich hinsichtlich ihrer Geometrie, ihres äußeren und inneren Durchmessers, der Porosität und der Porengröße auf beliebige Zellspezies anpassen, wodurch sich das erfindungsgemäße Reaktormodul für eine Vielzahl von Anwendungen anbietet. Überdies stellt die keramische Hohlfaser eine mit vielen verschiedenen Verfahren physikalischer und elektrischer oder chemischer Art modifizierbare Oberfläche zur Verfügung. Dadurch lässt sich eine verbesserte Immobilisierung der Zellen erreichen. Schließlich weisen keramische. Hohlfasern Poren auf, die eine Abfuhr von Stoffwechselprodukten und gegebenenfalls auch Zufuhr von Nährstoffen ermöglichen.

Erfindungsgemäß ist vorgesehen, dass die zu immobilisierenden Zellen mit den keramischen Hohlfasern in Kontakt gebracht werden, auf der Oberfläche der Hohlfasern aufwachsen, proliferieren und eine Monolayer bilden. Die von den immobilisierten Zellen abgegebenen toxischen Stoffwechselprodukte können durch die in den Hohlfasern enthaltenen definierten Poren in das Innere der Hohlfasern gelangen und von dort abtransportiert werden, oder über Nährstoffe aus dem Inneren der Hohlfaser über die definierten Poren versorgt werden.

Im Zusammenhang mit der vorliegenden Erfindung wird unter einer biologischen Zelle die Bau- und Funktionseinheit der Organismen verstanden, die durch ihre Fähigkeit zu Wachstum, Vermehrung und Stoffwechsel gekennzeichnet ist. Derartige Zellen können eukaryotische Zellen, wie tierische, pflanzliche oder Hefezellen sein. Im Zusammenhang mit der vorliegenden Erfindung werden unter Zellen auch prokaryotische Zellen, wie Bakterienzellen verstanden. In besonders vorteilhafter Ausgestaltung sind die Zellen menschliche oder tierische Zellen, insbesondere Leberzellen, Fibroblasten, Bindegewebszellen, Darmzellen, Blutzellen, Immunzellen, Hautzellen, Milzzellen, Nierenzellen oder Bauchspeicheldrüsenzellen. Die Zellen können natürlicherweise vorkommende oder gentechnisch manipulierte Zellen sein. Die Zellen können gesund oder krank sein, zum Beispiel immortalisiert oder carcinogen sein. Die Zellen können differenziert oder dedifferenziert, omnipotent, pluripotent oder unipotent sein.

Im Zusammenhang mit der vorliegenden Erfindung wird unter einer Immobilisierung ein räumliches Fixieren verstanden, insbesondere räumliches Fixieren der mindestens einen biologischen Zelle auf oder an der Oberfläche der keramischen Hohlfaser. Die Immobilisierung kann reversibel oder irreversibel sein. Sie kann durch einfaches Kultivieren und Anwachsen auf der Oberfläche der Hohlfaser durchgeführt werden, sie kann aber auch durch chemische oder physikalische Verfahren bewirkt oder beschleunigt werden.

Im Zusammenhang mit der vorliegenden Erfindung wird unter einer keramischen Hohlfaser eine aus keramischen Werkstoffen hergestellte Hohlfaser verstanden, also eine aus anorganischen und überwiegend nicht-metallischen Verbindungen oder Elementen aufgebaute Hohlfaser, die vorzugsweise zu mehr als 30 Volumen-%, kristalline Materialien enthält. Erfindungsgemäß können sowohl oxidische als auch nicht-oxidische keramische Materialien verwendet werden. Derartige nicht-oxidische Materialien können zum Beispiel Siliciumcarbid SiC oder Siliciumnitrid Si₃N₄ sein, die beispielsweise durch Pyrolyse von Polycarbosilanen beziehungsweise Polysilazanen hergestellt werden können. Selbstverständlich ist es auch möglich, oxidische keramische Membranen in Hohlfasergeometrie herzustellen, wobei zum Beispiel ein keramisches Pulver mit einem Binder vermengt und diese pastöse Masse extrudiert wird. Anschließend wird gesintert, wobei eine dichte Grünfaser in keramischer Struktur entsteht, deren Porengröße in Abhängigkeit verschiedener Parameter variiert werden kann, wie die Menge und die Natur des ausgebrannten Binders, dem Sinterregime, der Pulvermorphologie etc..

Es kann auch vorgesehen sein, keramische Materialien mit Cellulose als Binder zu verwenden, wobei diese mit einem Lösungsmittel, zum Beispiel N-Methyl-Morpholin-N-Oxid (NMMNO), in Lösung gebracht wird. Diese Cellulose-Lösung wird mit Keramikpulver vermischt und in an sich bekannter Weise weiterverarbeitet (DE 4426966 TITK vom 1.2.96).

Die Erfindung betrifft in einer weiteren Ausführungsform ein vorgenanntes Reaktormodul, wobei die kapillaren Membranen einen Innendurchmesser von 0,1 bis 4 mm, vorzugsweise 0,5 bis 1 mm, aufweisen. In einer weiteren Ausführungsform beträgt die Porengröße der in dem erfindungsgemäßen Reaktormodul eingesetzten Hohlfaser 0,05 bis 1 µm, vorzugsweise 0,1 bis 0,5 µm.

In einer weiteren Ausführungsform der vorliegenden Erfindung wird die Oberfläche der Hohlfasern modifiziert. Die erfindungsgemäß vorgesehene Modifizierung erlaubt eine verbesserte Immobilisierung der Zellen in vitro. Die Oberflächenmodifizierung kann mittels chemischer und physikalischer, insbesondere thermischer oder elektrischer Verfahren durchgeführt werden. So kann vorgesehen sein, chemische Verfahren wie radikalische oder ionische Pfropfcopolymerisationen, einfache Beschichtungen oder Gasphasenverfahren, wie die Niederdruck- oder Niedertemperatur-Plasmatechnik, einzusetzen. Besonders bevorzugt ist es dabei, Niederdruck-Plasmaverfahren einzusetzen, weil sich dadurch sowohl physikalische Eigenschaften der Oberfläche, wie Rauhigkeit, als auch die chemische Zusammensetzung der Oberfläche in einem Prozeßschritt definiert verändern lassen. Die erfindungsgemäß besonders bevorzugte Plasmabehandlung erlaubt eine freie Wahl des Substratmaterials, eine definierte Einstellung der Oberflächeneigenschaften ohne Veränderung der Volumencharakteristik, verbraucht nur wenig Chemikalien aufgrund des physikalischen Vakuumprozesses und erlaubt die Ausführung des Verfahrens in einem trockenen, geschlossenen System. Plasma, also ein ionisiertes Gas mit exakt gleicher Anzahl positiver und negativer Ladungen, lässt sich für die Ionisierung über einen sehr breiten Bereich von Drücken und Temperaturen einsetzen. Gemäß einer besonders bevorzugten Ausführungsform der vorliegenden Erfindung wird zur Oberflächenmodifizierung Niederdruckplasma mit einem Druck von 0,01 bis 1 mbar eingesetzt. Die Plasma-Atmosphäre besteht aus freien Elektronen, Radikalen, Ionen, UV-Strahlen und einer großen Anzahl unterschiedlich angeregter Teilchen. Durch die Wahl der Konzentration der chemischen Zusammensetzung der in den Gasraum eingeführten Monomere, die Verweilzeit der Molekel im Reaktionsraum, die eingetragene Hochfrequenzleistung und die elektrostatische Aufladung des Behandlungsguts kann die Ausbildung der neuen Oberfläche definiert bestimmt werden (Strobel et al., Plasma surface modification of polymers: Relevance to adhesion, Utrecht (1994)). Die erfindungsgemäß vorgesehene Behandlungszeit kann je nach Anforderung variiert werden, beträgt jedoch in einer besonders bevorzugten Ausführungsform eine Sekunde bis 10 Minuten, vorzugsweise 4 Sekunden bis 5 Minuten. Mittels der erfindungsgemäß vorgesehenen Niederdruck-Plasmatechnik kann die Oberfläche gereinigt und ein Ätzabtrag erreicht werden, kann die Mikrorauhigkeit verändert werden, kann die Ausbildung von Radikalstellen und anschließenden Sekundärreaktionen, wie zum Beispiel Vernetzungen oder Pfropfcopolymerisationen, ebenso wie Plasmapolymerisationen und damit die Ausbildung von homogenen haftfesten Filmen im Nanometerbereich erreicht werden.

Erfindungsgemäß ist es in einer bevorzugten Ausgestaltung auch vorgesehen, die Oberfläche mit funktionellen Gruppen, zum Beispiel hydrophoben, anionischen, kationischen, zum Beispiel Aminogruppen, oder polaren Gruppen und/oder mit Polypeptiden oder Proteinen zu versehen. Hierzu gehören vor allem Matrixproteine wie zum Beispiel Fibronectin, Laminin oder Kollagen.

Erfindungsgemäß kann vorgesehen sein, die Oberfläche der Hohlfasern mit Adhäsionspromotoren, wie Integrinen, Fibronectin, Vitronectin und/oder Kollagen zu versehen, um die Immobilisierung der Zellen zu erleichtern.

In einer besonders bevorzugten Ausführungsform der vorliegenden Erfindung kann vorgesehen sein, die Oberfläche mit einer Primerschicht zu versehen, die beispielsweise SiOx-Stöchiometrie aufweist. Dies stellt eine Verbindungsschicht dar - von organisch bis anorganisch, von Silicon bis Glas - an der sehr leicht weitere Gruppen gebunden werden können. Diese Primerschicht kann dann mit stickstoffhaltigen Gruppen, zum Beispiel mit funktionellen Gruppen, insbesondere Aminogruppen funktionalisiert werden. Denkbar ist es erfindungsgemäß auch, andere Oberflächen, insbesondere kationische Oberflächen, vorzusehen. Selbstverständlich kann die Primerschicht über Spacer oder Linker mit den funktionellen Gruppen verbunden werden.

In einer weiteren Ausführungsform der vorliegenden Erfindung sind die keramischen Hohlfasern parallel zueinander in einer Ebene in einem Rahmen angeordnet, wobei die Hohlfasern in Reihe miteinander verbunden oder getrennt voneinander vorliegen können. Der Rahmen kann aus Kunststoff, zum Beispiel Polystyrol, Polymethylmethacrylat, Polycarbonat oder Polypropylen, bestehen, oder dieses in wesentlichen Anteilen enthalten. In einer besonderen Ausführungsform der vorliegenden Erfindung ist das erfindungsgemäße Reaktormodul in Form einer, vorzugsweise quadratischen oder rechteckigen, Scheibe ausgeführt. Durch das erfindungsgemäß vorgesehene Anordnen der Hohlfasern in einer Ebene, beispielsweise in Matten, kann die Gefahr des "tunneling" bei der Außenraumdurchströmung und damit eine Effizienzverminderung vermieden werden.

Die thermische Ausgleichselemente Können zum Beispiel aus Glasfaser- oder Kohlefasersträngen bestehen, die den zum Beispiel bei Sterilisationen oder dem Einfrieren auftretenden Wärmespannungen und den verwendeten verschiedenen Materialien Rechnung tragen.

In einer weiteren bevorzugten Ausführungsform der vorliegenden Erfindung betrifft diese auch einen Reaktor, umfassend mindestens ein vorgenanntes Reaktormodul, insbesondere in Scheibenform, und ein Gehäuse. Erfindungsgemäß kann vorgesehen sein, mehrere der vorzugsweise scheibenförmigen Reaktormodule in Lagen mit definierten Abständen zueinander anzuordnen, so dass durch eine Schichtung mit einer ausreichenden Zahl derartiger scheibenförmiger Reaktormodule eine benötigte Oberfläche zusammengestellt werden kann. Der Zu- und Abgang der strömenden Medien kann durch ein Anschluss-System, zum Beispiel wie es in herkömmlichen Elektrodialysemodulen verwendet wird, realisiert werden. In den Endstücken sind die entsprechenden Anschlüsse vorhanden, über die die einzelnen Kanäle, gebildet von den Aussparungen in den scheibenförmigen Reaktormodulen, versorgt werden können.

In einer bevorzugten Ausführungsform der vorliegenden Erfindung weisen die Rahmen der Module Öffnungen auf, die als Zufuhr- beziehungsweise Abfuhröffnungen für Stoffwechselprodukte beziehungsweise Nährstoffe dienen können. Gemäß der vorstehend beschriebenen Ausführungsform, in der mehrere Reaktormodule hintereinander in einem Reaktor angeordnet sind, bilden die Rahmen der Module gleichsam die Außenwand des Reaktors. Das Gehäuse weist in seinen terminalen Bereichen Zutritts- und Austrittsöffnungen für die durch den Reaktor durchgeführte Flüssigkeit, zum Beispiel Plasma, auf. Die in den Randbereichen des Rahmens angeordneten Zufuhr- und Abfuhröffnungen stellen je nach Orientierung der hintereinander angeordneten Rahmen zueinander Kanäle in der durch die Rahmen gebildeten Außenwand des Reaktors dar, durch die gezielt in bestimmte Bereiche des Reaktors eine Zufuhr beziehungsweise eine Abfuhr von Substanzen ermöglicht wird. Durch ein geeignetes Hintereinanderschalten von mehreren Reaktormodulen können also Versorgungs- und Entsorgungskanäle für den Reaktorinnenraum, der sowohl den extra- als auch den intrakapillären Raum umfasst, gebildet und so eine funktionelle und räumliche Kompartimentierung ermöglicht werden. So können hintereinander in Reihe geschaltet verschiedene Kultivierungsbedingungen für die gleichen oder verschiedenen Zellen geschaffen werden, um so eine möglichst effiziente und vielfältig regelbare Organfunktion zu ermöglichen.

In einer besonders bevorzugten Ausführungsform der vorliegenden Erfindung weist der Reaktor weiterhin Oxygenator-, Wärmetauscher- und/oder Probeentnahmeeinheiten beziehungsweise Beimpfungszugänge auf. In einer besonders bevorzugten Ausführungsform der vorliegenden Erfindung wird der Reaktorinnenraum über im Bereich der terminalen Bereiche angeordnete Sterilfilter von der Umgebung abgeschlossen, um ein Ausschwemmen von Zellen und Zellbestandteilen in den Patientenkreislauf zu verhindern.

Der erfindungsgemäße Reaktor kann beispielsweise zum Aufbau einer künstlichen Leber eingesetzt werden, wobei der Reaktor zusammen mit Pumpen und Plasmaseparatoren in einen extrakorporalen Kreislauf, an dem auch der Patient beteiligt ist, angeschlossen wird. In einer solchen bevorzugten Ausführungsform wird dem erfindungsgemäßen Reaktor ein Plasmaseparator vorgeschaltet oder in diesen integriert, um einen zellfreien Betrieb des Reaktors zu ermöglichen.

Weitere vorteilhafte Ausgestaltungen der Erfindung ergeben sich aus den Unteransprüchen.

Die Erfindung wird anhand des folgenden Beispiels und der zugehörigen Figuren näher erläutert.

Die Figuren zeigen:
- Figur 1: ein scheibenförmiges Reaktormodul,
- Figur 2: eine bevorzugte Anordnung verschiedener Reaktormodule hintereinander,
- Figur 3: schematisch den Aufbau eines Reaktors im Längsschnitt und
- Figur 4: schematisch den Aufbau eines künstlichen Organs.

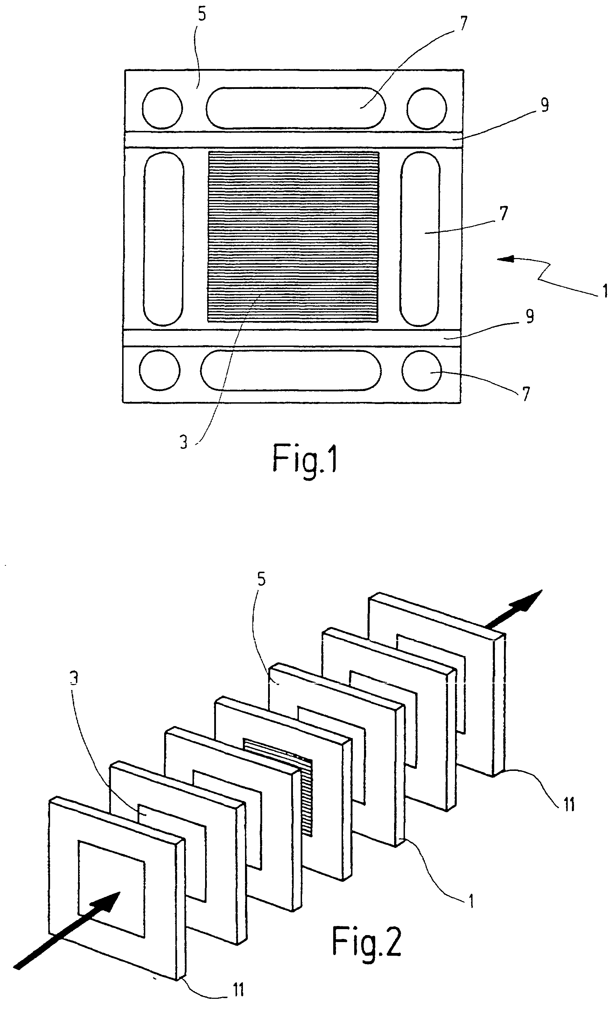

Die Figur 1 zeigt ein Reaktormodul 1, umfassend eine Vielzahl von in einer Ebene und parallel zueinander angeordneter keramischen Hohlfasern 3, die in einen quadratischen, scheibenförmigen Rahmen 5 eingespannt sind. Der Rahmen 5 umschließt also die in einer Ebene angeordneten Hohlfasern 3 allseits und hält sie räumlich fixiert. Der Rahmen umschließt zweidimensional gleichsam einen Innenraum, der die keramischen Hohlfasern 3 enthält und durch diese in jeweils einen außerhalb, und innerhalb der Hohlfasern 3 liegenden Raum getrennt wird. In den Rahmen 5 sind Öffnungen 7 für die Zu- und Abfuhr von nicht dargestellten Flüssigkeiten und Gasen integriert. Sowohl der Raum innerhalb der Hohlfasern 3 als auch außerhalb der Hohlfasern steht in Fluidverbindung mit den Zufuhr- beziehungsweise Abfuhröffnungen 7, so dass gezielt Fluide, wie Gase oder vorzugsweise Flüssigkeiten, in oder aus dem Kapillarinnen- beziehungsweise -außenraum geleitet werden können. Im Rahmen 5 sind ferner thermische Ausgleichselemente 9 in Form von zwei sich parallel zueinander über die gesamte Breite des Reaktormoduls 1 erstreckenden Aussparungen vorgesehen, in denen jeweils ein Kompensationsstreifen angeordnet ist.

Die Hohlfasern 3 wurden wie folgt hergestellt und oberflächenmodifiziert: N-Methyl-Morpholin-N-Oxid wurde als 50%ige Lösung in Wasser angesetzt, in die Zellulose gegeben wurde; die Zellulose wurde dispergiert und danach ein Teil des Wassers vakuumdestilliert. Die so erhaltene Suspension wird homogenisiert und es erfolgte die Zugabe von in N-Methyl-Morpholin-N-Oxid suspendiertem Keramikpulver. Anschließend wurde das Restwasser abdestilliert und die gesamte Suspension wurde noch einmal homogenisiert und entgast, wodurch eine homogene Spinnmasse erhalten wurde. In einem anschließenden Spinnprozess werden gequollene Fasern mit stabiler Hohlstruktur gewonnen. Während des Spinnprozesses wird die homogene Spinnmasse in ein Fällbad überführt, wodurch es zu einer Phaseninversion der Zellulose kommt, die von einer Stabilisierung der Hohlstruktur begleitet ist. Während des Quellvorganges erfolgt ein Austausch von Wasser gegen N-Methyl-Morpholin-N-Oxid. Die während des Spinnprozesses gewonnene gequollene Faser mit stabilisierter Hohlstruktur wird getrocknet, wodurch eine getrocknete Grundfaser gewonnen wird, die gesintert und die Hohlfaser 3 erhalten wird.

Die Hohlfasern 3 wurden mit einer Biomatrix - bevorzugt Kollagen - beschichtet. Anschließend wird eine Zellsuspension aus Hepatocyten auf die Zellträgerrahmen gegeben. Durch leichtes Bewegen der Rahmen kommen die Zellen in Kontakt mit den Hohlfasern und adhärieren an den Hohlfasern. Danach kann noch eine zweite Schicht Kollagen über die Zellen gelegt werden. Diese so mit Hepatocyten bestückte Rahmen können zur Langzeitlagerung in Flüssigstickstoff kryokonserviert werden.

Die Figur 2 zeigt schematisch eine mögliche Anordnung der Reaktormodule 1. Die Reaktormodule 1 werden in parallel zueinander liegenden Ebenen hintereinander angeordnet, so dass eine effiziente Durchströmung mit der nicht dargestellten Körperflüssigkeit des Patienten in Pfeilrichtung erfolgen kann. Dargestellt sind auch den erfindungsgemäßen Reaktormodulen 1 vor- und nachgeschaltete Sterilfiltrationsmodule 11, die ein Ein- und Ausschwemmen von Zellen und Zellbestandteilen verhindern.

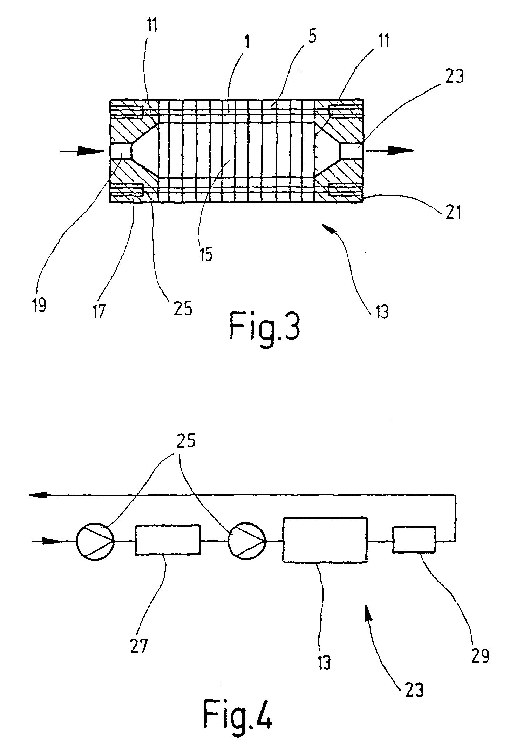

Die Figur 3 zeigt einen erfindungsgemäßen Reaktor 13. Der Reaktor 13 umfasst in hintereinander parallel zueinander angeordneten Ebenen scheibenförmige Reaktormodule 1, wobei der Rahmen 5 der Reaktormodule 1 jeweils gleichsam die Reaktoraußenwand bildet, die Zu- und Abfuhröffnungen (nicht dargestellt) Kanäle in der Reaktoraußenwand bilden und die keramischen Hohlfasern im Reaktorinnenraum 15 angeordnet sind. Je nach Ausführung der Zu- und Abfuhröffnungen 7 in den eingesetzten Reaktormodulen 1 und deren Anordnung im Reaktor 13 können gezielt voneinander weitgehend getrennte, hintereinanderliegende Kompartimente geschaffen werden, die sich durch unterschiedlichen Zellbesatz und/oder Kultivierungsbedingungen auszeichnen. Das Plasma des Patienten strömt durch eine in einem Reaktorendteil, also einen terminalen Bereich 17 angeordnete Zutrittsöffnung 19 in den Reaktor 13 ein, passiert einen Sterilfilter 11 und anschließend die keramischen Hohlfasern der Reaktormodule 1, um durch ein weiteres Sterilfiltrationsmodul 11 durch ein im gegenüberliegenden Teil des Reaktors angeordnetes Reaktorendteil 21 und die darin angeordnete Austrittsöffnung 23 auszuströmen.

Die Figur 4 stellt eine künstliche Leber 23 dar, umfassend Pumpen 25, einen Plasmaseparator 27, einen erfindungsgemäßen Reaktor 13 und einen Wärmetauscher 29. Das Blut eines nicht dargestellten Patienten wird durch die Pumpen 25 in Strömung gebracht und durch einen Plasmaseparator 27 geleitet, in dem Blutzellen abgetrennt werden. Das so erhaltene Plasma strömt durch den erfindungsgemäßen Reaktor 13, in dem die auf die keramischen Hohlfasern immobilisierten Hepatozyten ihre metabolische Funktion durchführen. Anschließend strömt das Plasma durch den Wärmetauscher 29 zurück in den Patienten. Der Wärmetauscher 29 kann ebenso wie der Plasmaseparator 27 gegebenenfalls im Reaktor 13 vorliegen.

## Patentansprüche

1. Reaktormodul umfassend mindestens eine keramische Hohlfaser (3) und mindestens eine biologische Zelle, wobei die mindestens eine keramische Hohlfaser (3) in einem Rahmen (5) aus Kunststoff eingespannt ist, wobei in dem Rahmen (5) des Reaktormoduls (1) thermische Ausgleichselemente (9) integriert sind und wobei die mindestens eine biologische Zelle auf der mindestens einen keramischen Hohlfaser (3) immobilisiert ist.

2. Reaktormodul nach Anspruch 1, wobei die biologische Zelle eine Leberzelle ist.

3. Reaktormodul nach einem der vorhergehenden Ansprüche, wobei die Oberfläche der Hohlfaser (3) modifiziert ist.

4. Reaktormodul nach einem der vorhergehenden Ansprüche, wobei die Oberfläche der Hohlfaser (3) chemisch oder physikalisch modifiziert ist.

5. Reaktormodul nach einem der vorhergehenden Ansprüche, wobei die Oberfläche der Hohlfaser (3) durch Einsatz eines Niederdruck-Plasmaverfahrens modifiziert ist.

6. Reaktormodul nach einem der vorhergehenden Ansprüche, wobei die Oberfläche der Hohlfaser (3) Adhäsionspromotoren, insbesondere Integrine, Fibronectin oder Kollagen aufweist.

7. Reaktormodul nach einem der vorhergehenden Ansprüche, wobei das Reaktormodul (1) in Form einer Scheibe ausgeführt ist.

8. Reaktormodul nach einem der vorhergehenden Ansprüche, wobei die Hohlfaser (3) einen Innendurchmesser von 0,1 bis 4 mm aufweist.

9. Reaktormodul nach einem der vorhergehenden Ansprüche, wobei die Porengröße der Hohlfaser (3) 0,05 bis 1 µm beträgt.

10. Reaktormodul nach einem der vorhergehenden Ansprüche, wobei in dem Rahmen (5) des Reaktormoduls (1) Zufuhr- und/oder Abfuhröffnungen (7) integriert sind.

11. Reaktor, umfassend mindestens ein Reaktormodul (1) nach einem der Ansprüche 1 bis 10 in einem Gehäuse (25).

12. Reaktor nach Anspruch 11, umfassend mindestens eine Oxygenator-, Wärmetauscher- und/oder Probeentnahme- beziehungsweise Befüllungseinheit.

13. Reaktor nach einem der Ansprüche 11 bis 12, wobei die Reaktormodule (1) in durch die Anordnung der Zufuhr- und Abfuhröffnungen definierten Kompartimenten angeordnet sind.

14. Reaktor nach einem der Ansprüche 11 bis 13, wobei in den Kompartimenten unterschiedliche Zellen vorhanden sind.

15. Reaktor nach einem der Ansprüche 11 bis 14, wobei in den Kompartimenten unterschiedliche Kultivierungsbedingungen vorliegen.

## Claims

1. Reactor module comprising at least one ceramic hollow fibre (3) and at least one biological cell, wherein the at least one ceramic hollow fibre (3) is fixed in a frame (5) made of plastic, wherein thermal compensating elements (9) are integrated in the frame (5) of the reactor module (1) and wherein the at least one biological cell is immobilised on the at least one ceramic hollow fibre (3).

2. Reactor module according to Claim 1, wherein the biological cell is a liver cell.

3. Reactor module according to one of the preceding claims, wherein the surface of the hollow fibre (3) is modified.

4. Reactor module according to one of the preceding claims, wherein the surface of the hollow fibre (3) is chemically or physically modified.

5. Reactor module according to one of the preceding claims, wherein the surface of the hollow fibre (3) is modified by using a low-pressure plasma process.

6. Reactor module according to one of the preceding claims, wherein the surface of the hollow fibre (3) has adhesion promoters, in particular integrins, fibronectin or collagen.

7. Reactor module according to one of the preceding claims, wherein the reactor module (1) is configured in the form of a disc.

8. Reactor module according to one of the preceding claims, wherein the hollow fibre (3) has an inside diameter of 0.1 to 4 mm.

9. Reactor module according to one of the preceding claims, wherein the pore size of the hollow fibre (3) amounts to 0.05 to 1 µm.

10. Reactor module according to one of the preceding claims, wherein feed and/or discharge openings (7) are integrated in the frame (5) of the reactor module (1).

11. Reactor comprising at least one reactor module (1) according to one of Claims 1 to 10 in a housing (25).

12. Reactor according to Claim 11 comprising at least one oxygenator, heat exchanger and/or sample-taking or filling unit.

13. Reactor according to one of Claims 11 to 12, wherein the reactor modules (1) are arranged in compartments defined by the arrangement of the feed and discharge openings.

14. Reactor according to one of Claims 11 to 13, wherein different cells are present in the compartments.

15. Reactor according to one of Claims 11 to 14, wherein different culture conditions are present in the compartments.

## Revendications

1. Module réacteur comprenant au moins une fibre creuse céramique (3) et au moins une cellule biologique, cette au moins une fibre creuse céramique (3) étant tendue dans un cadre (5) en matière plastique, dans lequel des éléments de compensation thermique (9) sont intégrés, dans le cadre (5) du module réacteur (1) et au moins une cellule biologique est immobilisée sur l'au moins une fibre creuse céramique (3).

2. Module réacteur selon la revendication 1,
**caractérisé en ce que**
la cellule biologique est une cellule de foie.

3. Module réacteur selon l'une quelconque des revendications précédentes,
**caractérisé en ce que**
la surface de la fibre creuse (3) est modifiée.

4. Module réacteur selon l'une quelconque des revendications précédentes,
**caractérisé en ce que**
la surface de la fibre creuse (3) est modifiée chimiquement ou physiquement.

5. Module réacteur selon l'une quelconque des revendications précédentes,
**caractérisé en ce que**
la surface de la fibre creuse (3) est modifiée par la mise en oeuvre d'un procédé de plasma basse pression.

6. Module réacteur selon l'une quelconque des revendications précédentes,
**caractérisé en ce que**
la surface de la fibre creuse (3) présente des promoteurs d'adhésion, notamment des intégrines, la fibronectine ou des collages.

7. Module réacteur selon l'une quelconque des revendications précédentes,
**caractérisé en ce que**
le module réacteur (1) présente la forme d'une plaquette.

8. Module réacteur selon l'une quelconque des revendications précédentes,
**caractérisé en ce que**
la fibre creuse (3) présente un diamètre intérieur de 0,1 à 4 mm.

9. Module réacteur selon l'une quelconque des revendications précédentes,
**caractérisé en ce que**
la grosseur des pores de la fibre creuse (3) est de 0,05 à 1 µm.

10. Module réacteur selon l'une quelconque des revendications précédentes,
**caractérisé en ce que**
des ouvertures d'alimentation et/ou d'évacuation (7) sont intégrées dans le cadre (5) du module réacteur (1).

11. Réacteur, comprenant au moins un module réacteur (1) selon l'une quelconque des revendications 1 à 10, dans un boîtier (25).

12. Réacteur selon la revendication 11, comprenant au moins une unité d'oxygénateur, d'échangeur thermique et/ou et de prise d'échantillon ou de remplissage.

13. Réacteur selon l'une quelconque des revendications 11 à 12,
**caractérisé en ce que**
les modules réacteurs (1) sont disposés dans des compartiments définis par la disposition des ouvertures d'alimentation et d'évacuation.

14. Réacteur selon l'une quelconque des revendications 11 à 13,
**caractérisé en ce que**
les compartiments contiennent des cellules différentes.

15. Réacteur selon l'une quelconque des revendications 11 à 14,
**caractérisé en ce que**
les compartiments présentent des conditions de culture différentes.
